Europäisches Patentamt

European Patent Office

Office européen des brevets

(11) Publication number: **0 221 226**
**A1**

## (12) EUROPEAN PATENT APPLICATION

(21) Application number: **85850342.8**

(22) Date of filing: **25.10.85**

(51) Int. Cl.⁴: **G01N 33/34** , G01N 3/00

(43) Date of publication of application:
**13.05.87 Bulletin 87/20**

(84) Designated Contracting States:
**AT BE CH DE FR GB IT LI LU NL SE**

(71) Applicant: **Varila, Reijo**
**Maneesinkatu 40 B 50**
**SF-00170 Helsinki(FI)**
Applicant: **Heinonen, Aimo**
**Örkkiniityntie 19**
**SF-02970 Espoo(FI)**

(72) Inventor: **Varila, Reijo**
**Maneesinkatu 40 B 50**
**SF-00170 Helsinki(FI)**

(74) Representative: **Kummelsten, Per-Arne et al**
**UPPSALA PATENTBYRA Box 9039**
**S-750 09 Uppsala(SE)**

(54) **A method and appliance for handling and testing paper.**

(57) A method for the mechanical testing of paper, wherein a test stripe (2) cut of a paper web is transferred a certain distance (b) past a sheet cutter (12) which has been arranged to cut a sample sheet (3) and feasibly a test sample (4, 5, 6) from the sheet and the sample is transferred to a paper testing device such as a tensile strength tester (21), a tear tester (22) or alternatively to a burst tester, and tested.

A paper testing appliance, which includes a sheet cutter (12) for cutting the sheets (3) as well as a paper tester such as a tensile strength tester (21), a tear tester (22) and/or a burst tester (23).

**Fig. 1 a**

**Fig. 1**

EP 0 221 226 A1

Xerox Copy Centre

# A METHOD AND APPLIANCE FOR HANDLING AND TESTING PAPER

## BACKGROUND OF THE INVENTION

The invention relates to a method for handling and testing paper, as defined in the introductory part of claim 1, by an appliance which tests paper. Another object of the invention is an appliance for handling and testing paper, as defined in the introductory part of claim 3.

Nowedays the testing and especially the handling of paper takes place manually in a laboratory. Thus the specimen sheets as well as the test samples have to be cut mainly manually from the specimen sheets from the paper web. In addition, the attachment of the test samples to the actual tester, for example between the jaws of a tester measuring the tensile strength of the paper, takes place manually.

The manual handling and testing paper is first and foremost expensive due to the generally expensive manual labour and the social and other cost brought up by such labour. Moreover, the manual cutting of the specimen sheets and the test samples is inaccurate and the experimental results depend often on the prudence and accuracy of the handler. In addition, the actual testing of paper by testers, such as a tensile strength tester, a tear tester measuring resistance to tearing and a burst tester measuring bursting strength, is also inaccurate and scattering can be detected in the test results.

## SUMMARY OF THE INVENTION

An object of the present invention is to eliminate the drawbacks mentioned above and to provide a method for handling and testing paper by an appliance which tests paper so that the handling and testing of paper takes place essentially fully mechanically.

A further object or the invention is to provide a method for handling and testing paper in order to obviate the scattering of test results which depends on the handler, so that the results will be more comparable with each other than before.

A further object of the invention is to provide an appliance for handling and testing paper from a sample strip of paper cut of a paper web.

Regarding the characteristic features of the invention reference is made to the claims.

The invention is based upon the handling of the stripe cut of the paper web, i.e. the cutting and subsequent mechanical testing which are done fully automatically. According to the invention a test stripe is cut of the paper web running on a paper machine, or from a web in storage, and fed into a tester which first cuts it into a certain width and into specimen sheets. The specimen sheets are then cut according to a program into test samples for the desired tests, and the test samples are transferred to the actual testers. Lastly the testers in question test the samples according to a program and give the experimental results.

The appliance according to the invention preferably includes a side-cutting plier for cutting the stripe cut out of the paper web into a sample stripe of desired width, as well as a sheet cutter for cutting the specimen sheets and a test sample cutter for cutting the actual test samples. The side-cutting plier, the sheet and test sample cutters include transferring devices for transferring said stripe and sheet as well as, lastly, for transferring the test samples from one cutter to another and correspondingly to the actual paper tester.

The control of the handling and testing preferably takes place by a programmable central control unit which is arranged to control the cutting of the test stripe and the cutting of the sheets cut of the stripe as well as the cutting of test samples. The control unit preferably also controls the turning of the sheets and/or the samples in order to effect the desired test direction with respect to the direction, in which the paper web is formed, and also the actual testing of the paper.

The central control unit can be arranged to cut various test samples, as well as test samples meant for the various testers, from the specimen sheets and to transfer the samples into the appropriate tester according to a predetermined program, for example a programmed number of test samples to each tester. In addition, the central control unit can be arranged to control the testing carried out by each tester so that a desired number of tests is accomplished, for example, many consecutive or feasibly parallel tests such as simultaneous tearing test made from the same sample by two or more pairs of clamps situated in parallel. Moreover, the programmable central control unit can be connected, for example, to the information and/or control system of a paper laboratory, or preferably even to the control room of the paper machine, for example, to a device indicating the tested magnitudes or even to control the paper manufacturing process according to the control parameters that effect the test magnitudes.

## DETAILED DESCRIPTION OF THE INVENTION

The invention is explained below in detail by the aid of examples refering to the inclosed drawings in which:

Fig. 1 shows a diagrammatic side elevation of one embodiment of the device according to the invention for handling and testing the tensile strength of paper,

Fig. 1a is a perspective view showing the paper sample, prepared according to Fig. 1, in a tensile strength tester,

Fig. 2 is a diagrammatic top plan view of another embodiment of the invention for handling and testing the paper by various testers,

Figs 3-4 show ways in which the paper can be torn according to some advantageous embodiments of the invention,

Figs. 5a-b are partly sectional side views of a clamp shown in an opened and a closed position respectively as one example of an application according to the invention.

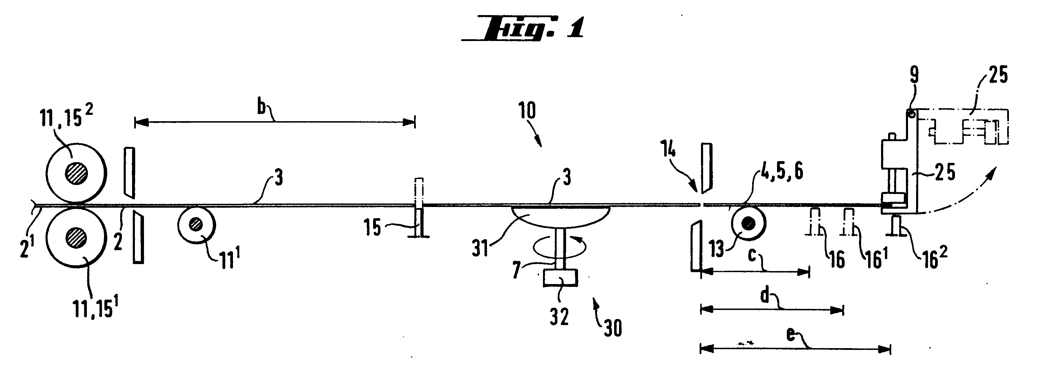

Fig. 1 shows a sidewise viewed diagram one embodiment of the method and device according to the invention, for handling and testing according to the invention, for handling and testing paper by a paper testing device 10. According to the performance diagram a stripe of a certain width is cut from the paper web in the direction of the motion of the paper web or in a cross-wise direction perpendicular to the direction of the motion of the web by a side-cutting plier 11. The side cutting plier consists, for example, of a lower cutting blade $15^1$ and an upper cutting blade $15^2$ which rotates against the lower blade. The blades form at the same time a transferring device for transferring the test stripe to the testing device. The stripe 2 cut according to fig. 1 is transferred in its lengthwise direction, for example in the direction perpendicular to the width of the paper web or, alternatively, in the direction of the webs motion a certain distance b past the sheet cutter 12 by, for example, the transferring device 11 mentioned above or by a separate transferring device $11^1$ such as a pair of rolls. The sheet cutter has been arranged to cut a sample sheet 3 after the stripe has been transferred through the above mentioned distance b. In the shown embodiment the above mentioned distance b, through which the above mentioned stripe is transferred, is determined by a back stop 15 arranged to rise up into the moving path of the test stripe for the duration of the time taken to cut the sheet.

The sheet 3 cut according to fig. 1 is turned, if so desired, through 90° relative to a vertical shaft 7 perpendicular to the sheet by a turning device 30 such as upwards opening suction pad 31 turned by a power unit 32, i.e. the sheet 3 is turned by a turning member, to which the sheet 30 is attached for the duration of the turning due to the underpressure formed in the suction pad.

After the feasible turning the sheet 3 is transferred by a transferring device 13, such as a pair of rolls through a distance c, d or e past the sample cutter 14 arranged to cut a test sample 4, 5 or 6 after the specimen sheet has been transferred through the distance c, d or e respectively. The above mentioned distance of transfer is determined by transferring the specimen sheet against a back stop 16, $16^1$ or correspondingly $16^2$. The backstop has been arranged to rise into the moving path of the specimen sheet, for example by the control of the central control unit for forming the various test samples for the various tests and testers.

In Fig. 1a the test sample 4 formed according to fig. 1 has been transferred into a tensile strength tester 21 between two parallel gripping members 25 pivoted on a shaft 9, i.e. the test sample is inserted from below between a tensioning member, i.e. the tensioning nails $25^2$, $25^3$ which are parallel to the gripping nails $25^1$ (see fig. 1a).

The tensioning nails $25^2$, $25^3$ have been arranged to move away with respect to each other in order to test the test sample 4 placed between them. The power unit for drawing the pairs of tensioning nails $25^2$, $25^3$ apart in the direction of the sample 4 and registering the tensioning force has not been shown in order to illustrate and clarify the principle involved. The force affecting the tensioning nails $25^2$ or feasibly the other tensioning nails $25^3$ is registered, according to the state of the art, for example by a tester called L & Testomatic 556; manufactured by AB Lorentzen & Wettre, or by other known testers. After the testing of the test sample 4, the nails $25^2$, $25^3$ open, the sample falls and the jaws move into their initial position.

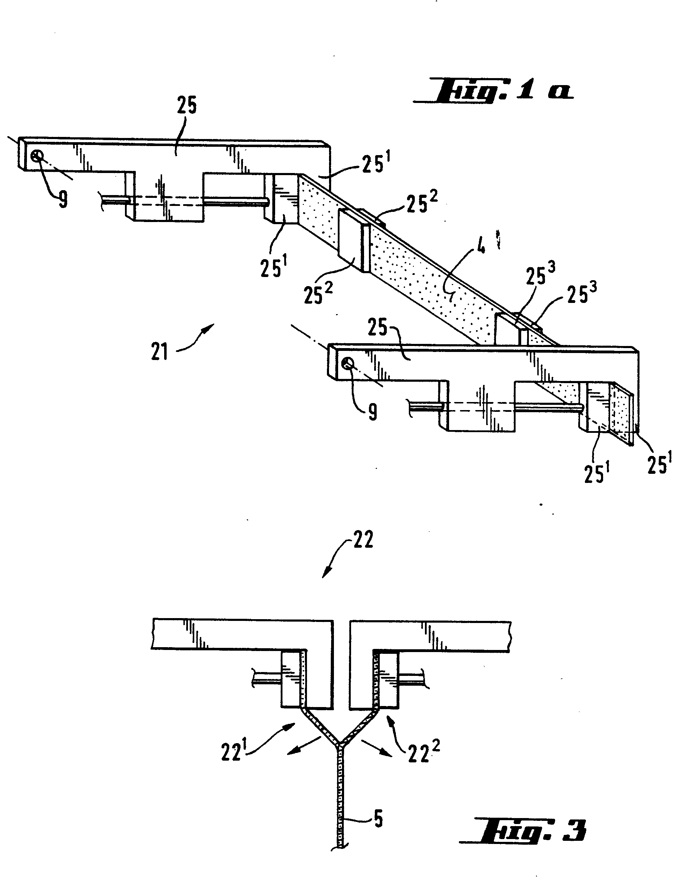

Parallel to the tensile stress tester 21, shown in fig. 1, other testers such as a tear tester or a burst tester can be arranged as shown in fig. 2.

Fig. 2 shows a method and device according to fig. 1 viewed from above. A test stripe 2 is cut from the ribbon like strip $2^1$ by a side-cutting plier 15, the sheet 3 is cut by the sheet cutter 12 and turned through 90° in the turning device 30 and cut into a test sample 4, 5 and/or 6 by the sample cutter 14 according to fig. 1. After passing by the sample cutter 14 the samples 4, 5 and/or 6 are transferred into the relevant testers i.e. to the tensile strength tester, the tear tester 22 and/or the burst tester by the transferring devices 17, $17^1$ and/or $17^2$ such as a pair of transferring rolls, transferring nails etc. Lastly, the testing is effected in the testers in a customary manner.

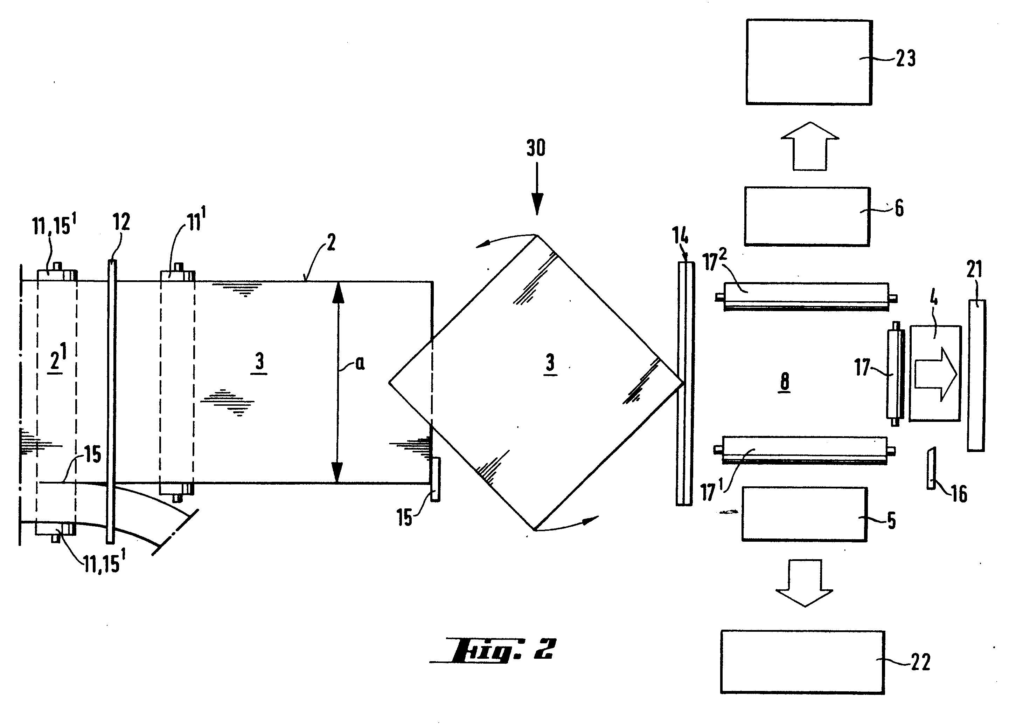

Fig. 3 shows the basic construction and functioning of a tear tester 22, according to the invention. The test sample has been placed between the nails of two parallel clamps 22', 22², which are operative by the aid of a pressurized medium, and the clamps are moved away from each other in a mainly perpendicular direction to the test sample's plane of transfer by the aid of a power unit, such as a trasfer motor, a cylinder actuated by a pressurized medium or other like device, at which the test sample 5 is torn, and during this event, for example, the instantaneous tearing force, the tearing force as a function of tearing time, the average tearing force and/or the energy used per tearing distance, can be registered in order to characterize the properties of the paper.

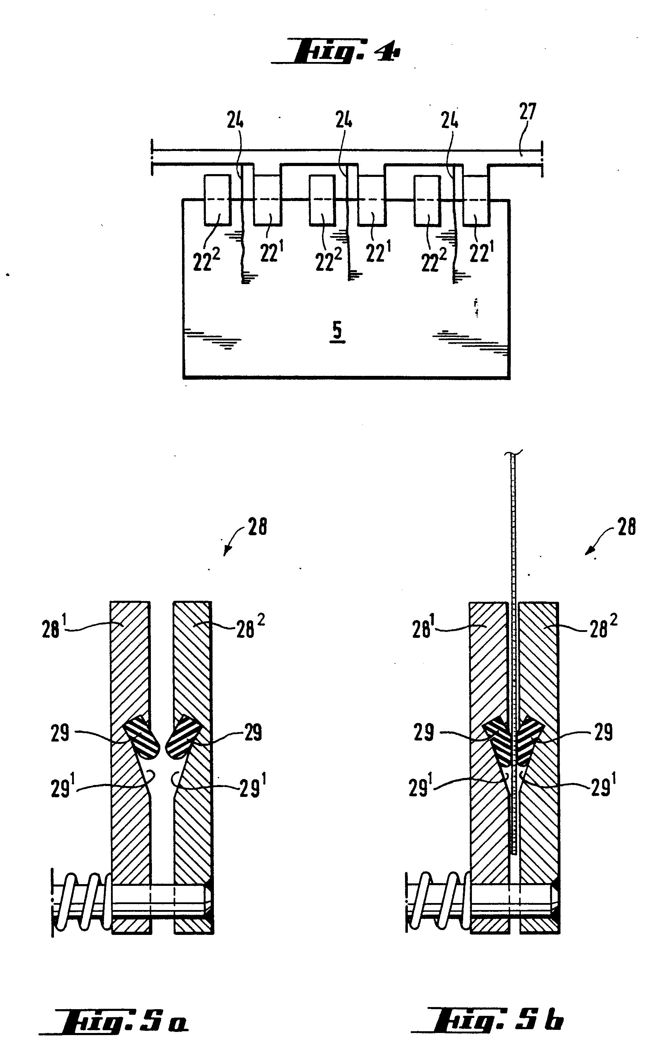

Fig. 4 shows a tear tester, according to another application of the invention, mainly as a basic drawing. The test sample 5 has been placed between a plurality of clamps 22', 22² situated in parallel according to fig. 2 so that the gripping nails are aligned. Afterwards the parallel clamps 22', 22² are moved away from each other according to fig. 3, at which the sample 5 is torn between the clamps which have been situated in pairs on the frame 27, from the initial incisions formed on the samples by blades 24 situated between the clamps 27.

Figs. 5a, 5b show the clamp of the tensile strength tester, presented for example in fig. 1, i.e. the gripping nail 28 in its open and closed positions, figs. 5a and 5b respectively. On the inner surfaces of the gripping nails 28', 28² which are pressed against each other, there are relatively low friction members 29 so that as the nails are initially pressed against each other the test sample is engaged first with the friction memebers 29. The friction members are manufactured of elastic material such as nailon, rubber or similar material. After the tensioning is started, the test sample is drawn away from between the nails, for example, by another gripping member, at which the sample can turn between the nails in the direction of the tensioning force. Only after the turning mentioned above, the nails are pressed firmly against each other, at which the tension subjected upon the sample is symmetrical.

The method and appliance according to the invention is intended for carrying out all the usual tests of strength made to paper. In conjunction with the method and appliance usual known methods and devices can be arranged for use. In the presented applications, for example, the transferring of the sample stripe, the specimen sheet and/or the test sample has been arranged to take place by an apron conveyor. Instead of these, as shown for example in fig. 1, a gripping member 25 pivoted round a shaft 9 and including a gripping clamp 25';

or a gripping jaw, member or device mov able by a power unit, such as a transferring motor or a piston operable by pressurized medium, can be used for transferring the above mentioned stripe, the specimen sheet and/or the test samples. In additon, a central control such as a programmable central control unit, can be used in connection with the method in order to control and operate the parts of the appliance as well as to effect the desired test program, at which the central control unit can be arranged to cut the sample stripe into the desired width and the sheets as well as samples into the desired size in the moving and/or cross-wise direction of the web. Thus, the central control unit operates all the operational members, such as the cutters, the transferring devices, the turning devices and the gripping members and so on. In additon, the central control unit can be arranged to control the actual testing, for example to carry out a desired number of tensile strength, tearing and bursting tests in a desired manner.

The intention of the examples is to illustrate the invention, without limiting it in any way. Thus, the various specimen sheets or test samples can be formed by moving the stripe 2 or correspondingly the sheet 3 a programmed distance, corresponding to the size of the sheet or sample, past the cutter by using a conveyor/transferer, a reel conveyor or the like conveying member, for example a conveying member equipped with gripping nails, and a motor, controlled by the control program. In addition, the transfer of the sample into the tear tester 22 can be effected by gripping members, of for example the type shown in figs 1, 1a which members have been mounted turnably upon a horizontal shaft; at which the griping members 25 with their gripping nails can be arranged to insert the sample into the compressive nails 22', 22² of the tearing tester, according to fig. 3 or 2, from the forming and handling site 8 of the samples (fig. 2). In additon, the transfer of the sample from the forming and handling site 8 into a burst tester 23, which is preferably included in the appliance can be effected similarly by using special gripping members 25 equipped with gripping nails 25', 25² in a manner presented above.

## Claims

1. A method for handling paper and testing it by a testing appliance (10), wherein a test stripe - (2) of a certain width (a) is cut of the paper web - (1), preferably in a cross-wise direction to the web and the stripe, is cut into specimen sheets (3) for strength tests, **characterized** in that the stripe (2) is transferred in a manner known per se lengthwise, ie. in the direction parallel to the width of the web, a certain distance (b) past a sheet cutter 12 which is arranged to cut a specimen sheet (3) after the stripe has been transferred through said distance (b), and that the cut specimen sheet is turned/left unturned through 90° round the shaft - (7) which is perpendicular to the sheet and the sheet is transferred a certain distance (c, d, e), in a manner known per se, by another transferring device (13) past a specimen cutter (14) which is arranged to cut a test sample (4, 5, 6) after the specimen sheet has been transferred through said distance (c, d, e), and that the test sample (4, 5, 6) cut into a said width (c, d, e) is transferred into a paper tester (21, 22, 23), such as a tensile strength tester (21), a tear tester (22) or a burst tester (23), and is tested.

2. A method according to claim 1, wherein the test sample (5) is transferred into a tearing tester - (22), **characterized** in that the paper's resistance to tear is tested by placing the sample (5) between two parallel clamps (22', 22²) and by moving the clamps away from each other in a direction mainly perpendicular to the plane of the test sample (5) and registering the instantaneous tearing force, the tearing force as a function of tearing time, the average tearing force and/or the energy used per tearing distance.

3. A paper testing appliance (10) for handling paper and testing it by using a test stripe (2) which has been cut to a certain width from the paper web (1), **characterized** in that the appliance (10) includes: a sheet cutter (12) including a transferring device (11) known per se for cutting of specimen sheets (3) and transferring the stripe (2) through a certain distance (b) past the sheet cutter, for example, against a back stop (15), the cutter being arranged to cut a specimen sheet (3) as the stripe has been transferred through said distance; a turning device (30) arranged to turn the speciemen sheet through 90°, if this is desired, round a shaft - (7) perpendicular to the sheet in order to cut a test sample in a desired direction through the specimen sheet with respect to it, and correspondingly with respect to the web (1); a sample cutter (14) including a transferring device (13), for cutting test samples (4, 5, 6) and transferring the sheet (13) through a certain distance (c, d, e) past the sample cutter, for example, against a back stop (16), the sample cutter being arranged to cut a test sample as the sheet has been transferred through said distance (c, d, e); and a paper tester (21, 22, 23), such as a tensile strength tester (21), a tear tester - (22) and/or a burst tester (23) as well as a transferring member (24, 25, 26) for transferring the test sample (4, 5, 6) into said tester.

4. A paper testing appliance (10), according to claim 3, which includes a tear tester (2) with its transferring members, **characterized** in that the tear tester (2) includes two parallel clamps (22', 22²) arranged to receive the test sample (5), and that the clamps are arranged to move away from each other in a direction mainly perpendicular to the plane of the test sample and are equipped with devices for registrering and measuring the instantaneaous tearing force, the tearing force as a function of tearing time, the average tearing force and/or the energy used per tearing distance.

Fig. 1

0 221 226

**Fig: 1 a**

25

25¹

25²

25¹

25²

4

25³

25³

21

25

9

9

25¹

25¹

22

22¹

22²

5

**Fig: 3**

Fig. 2

## Fig. 4

## Fig. 5a     Fig. 5b

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 838 596 (R. NEUENSCHWANDER) * Abstract; figure 1 * | 1,3 | G 01 N 33/34 G 01 N 3/00 |
| A | US-A-3 640 162 (J.W. FLEMING, Jr. et al.) * Column 9, lines 52-61; figure 8 * | 1,3 | |
| A | DE-A-3 109 591 (BASF) * Page 1, lines 3-19; figures * | 1,3 | |
| A | DE-C- 694 497 (R. MARTIN) * Whole document * | 2 | |

-----

**TECHNICAL FIELDS SEARCHED (Int. Cl.4)**

G 01 N 3/00
G 01 N 33/00
G 01 N 1/00
D 21 G 9/00

The present search report has been drawn up for all claims

| Place of search | Date of completion of the search | Examiner |
|---|---|---|
| THE HAGUE | 12-06-1986 | ANTHONY R.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82